# EUROPEAN PATENT APPLICATION

(11) **EP 1 752 468 A1**
(43) Date of publication of application: **14.02.2007**
(21) Application number: 05743930.9
(22) Date of filing: 27.04.2005
(51) Int. Cl.: C07K 14/08, C12N 7/04, A61K 39/12

(54) **METHOD FOR THE PRODUCTION OF EMPTY VIRAL CAPSIDS IN YEASTS, SAID CAPSIDS COMPRISING PROTEINS DERIVED FROM PVP2 OF THE VIRUS THAT CAUSES INFECTIOUS BURSAL DISEASE (IBDV)**

(30) Priority: 30.04.2004 ES 200401044
(71) Applicant: Consejo Superior de Investigaciones Cientificas, E-28006 Madrid (ES); Bionostra S.L., 28760 Tres Cantos Madrid (ES)
(72) Inventor: RUIZ CASTÓN, José, Centro Nac. de Biotecnología, Campus de Cantoblanco, 28049 MADRID (ES); SAUGAR GÓMEZ, Irene, Centro Nac. de Biotecnologia, Campus de Cantoblanco, 28049 MADRID (ES); LUQUE BUZO, Daniel, Centro Nac. de Biotecnología, Campus de Cantoblanco, 28049 MADRID (ES); ABAITUA ELUSTONDO, Fernando, Centro Nac. de Bio., Campus de Cantoblanco, 28049 Madrid (ES); OÑA BLANCO, Ana M, Centro Nac. de Biotecnologia, Campus de Cantoblanco, 28049 MADRID (ES); GONZÁLEZ DE LLANO, Dolores, Centro Nac de Bioteth, Campus de Cantoblanco 2804 Madrid (ES); RODRÍGUEZ AGUIRRE, José F., Centro Nac de Biotech, Campus de Cantoblanco, 28049 Madrid (ES); RODRÍGUEZ FERNÁNDEZ-ALBA, Juan, Ramn, E-28760 TresCantos (MADRID) (ES)
(74) Representative: ABG Patentes, S.L.
(86) International application number: PCT/ES2005/070052
(87) International publication number: WO 2005/105834

(57) **Abstract**

The empty capsids of the infectious bursal disease virus (IBDV) are formed by the assembly of proteins derived from the pVP2 protein of IBDV, with a different size and with an application in producing vaccines and in preparing gene therapy vectors.

## Description

### FIELD OF THE INVENTION

The invention relates to a process for producing empty viral capsids consisting of proteins derived from the protein pVP2 (pVP2*) of the infectious bursal disease virus (IBDV). Said capsids can be used in producing vaccines and in preparing gene therapy vectors.

### BACKGROUND OF THE INVENTION

The infectious bursal disease virus (IBDV) is a member of the *Birnaviridae* family, which infects various avian species and is directly responsible for a severe immunosuppressive disease, causing economic losses in the avian industry worldwide (Sharma JM et al. 2000. infectious bursal disease virus of chickens: pathogenesis and immunosuppression. Developmental and Comparative Immunology 24:223-235; van den Berg TP et al. 2000. Infectious bursal disease (Gumboro disease). Revue scientifique et technique (International Office of Epizootics) 19:509-543).

IBDV particles are icosahedral with symmetry T=13, they lack the envelope and are formed by a single protein layer. Until now, the approaches aimed at obtaining an atomic model for IBDV particles have failed. For this reason, the available information on their structure is based on three-dimensional models generated from images obtained by electron cryomicroscopy of the purified virus and empty viral particles. Based on these studies, it has been observed that the outer surface of the particle is formed by a continuous lattice of 260 trimers of VP2 protein (37 kDa) arranged in five different conformations. The inner side of the particles contains 200 trimers of VP3 protein (29 kDa), the latter ones, independently of one another, are bound to the basal area of the VP2 trimers. It has been suggested that a third polypeptide, VP4 (28 kDa), could also be part of the particles, being located at the base of the pentamers forming the angles of the icosahedral structure.

Proteins VP2, VP3 and VP4 are produced from proteolytic processing of a precursor polypeptide (or polyprotein) with a size of 109 kDa. This precursor is auto-catalytically processed, releasing the polypeptides pVP2 (VPX), VP3 and VP4. The VP4 domain, which is located in the central region of the polyprotein, belongs to the family of Ion proteases and is responsible for the proteolytic cleavage. Polypeptides pVP2 and VP3 are directly responsible for assembling the capsids. The peptide pVP2 undergoes a last cleavage at its C-terminal end before giving rise to the mature form of the protein (VP2), which is the form found in the purified particles. This pVP2 processing is necessary for correctly forming the capsids and it requires the presence of VP3, although the protease responsible for it has not yet been identified. As is known, morphogenesis is a vital process for the viral cycle, requiring two successive steps associated with modifications in the precursor polypeptides. To that end, viruses have developed strategies which allow the correct and sequential interaction between each one of their components. One of these strategies, frequently used by icosahedral viruses, is the use of polypeptides of a single polyprotein as the basis of their structural components. In these cases, the appropriate protcolytic processing of said polyprotein plays a crucial role in the assembly process.

Conventional vaccines used for controlling infectious bursal disease are based on the use of strains, with different degrees of virulence, of the IBDV itself grown in cell culture or in embryonated eggs. The extracts containing the infectious material are subjected to chemical inactivation processes to produce inactivated vaccines or are used directly to produce live attenuated vaccines. This latter type of vaccines has the typical drawbacks associated with the use of live attenuated vaccines, specifically, the risk of mutations reverting the virulence of the virus or making it lose its immunogenicity.

Recombinant subunit vaccines containing the IBDV VP2 protein expressed in several expression systems, for example, bacteria, yeasts or baculovirus, usually in fusion protein form, have been disclosed. The results obtained in chicken immunization tests with said vaccines have not been completely satisfactory.

Virus-like particles (VLPs) constitute an alternative to the use of live attenuated vaccines and of recombinant subunit vaccines. VLPs are obtained by self-assembly of all or part of the subunits constituting the viral capsid, and they mimic the structure and antigenic properties of the native virion, although they lack genetic material, so they are unable to replicate themselves. Apart from their application for vaccination purposes, VLPs can be used as vectors of molecules of biological interest, for example, nucleic acids, peptides, proteins, etc.

The use of different IBDV gene expression vectors has allowed developing different systems of producing viral assemblies. In this sense, the production of several VLPs of IBDV by means of expression of the viral polyprotein using several expression systems, for example mammal cells (Fernández-Arias A et al. 1998. Expression of ORF A1 of infectious bursal disease virus results in the formation of virus-like particles. J. Gen. Virol. 79:1047-54) or alternative vectors based on the use of recombinant baculoviruses (rBVs) (Vakharia VN. 1997. Development of recombinant vaccines against infectious bursal disease. Biotechnology Annual Review 3;151-68; Kibenge FS et al. 1999. Formation of virus-like particles when the polyprotein gene (segment A) of infectious bursal disease virus is expressed in insect cells. Can J Vet Res 63:49-55), have been described.

The use of said viral vectors has allowed producing different types of VLPs in insect cells, including particles formed by the assembly of a recombinant VP2 protein, 20-30 nm in diameter (Min-Ying Wang et al. 2000. Self-Assembly of the infectious bursal disease virus capsid protein, rVP2, expressed in insect cells and purification of immunogenic chimeric rVP2H particles by immobilized metal-ion affinity chromatography. Biotechnology and Bioengineering. Vol. 67(1):104-111); VLPs with symmetry T=1 obtained by means of expressing a chimeric form of the VP2 protein (rVP2-456) (Martinez-Torrecuadrada JL et al. 2000. Different architectures in the assembly of infectious bursal disease virus capsid proteins expressed in insect cells. Virology 278:322-331); VLPs containing all the proteins present in the viral particle (Maraver A ct al. 2003. The oligomerization domain of VP3, the scaffolding protein of infectious bursal disease virus, plays a critical role for capsid formation. Journal of Virology 77:6438-49); and VLPs produced by the expression of a chimeric form of the viral polyprotein formed by the fusion of the latter to a heterologous protein for the purpose of improving VLPs formation efficiency (Chevalier C et al. 2002. The maturation process of pVP2 requires assembly of infectious bursal disease virus capsids. J. Virol. 76:2384-92).

The various processes for producing VLPs of IBDV hereinbefore described have several drawbacks which reduce or prevent their applicability in generating vaccines against IBDV given that:
- i): the vectors developed for the production of VLPs of IBDV are based on the use of recombinant viruses derived from the vaccine virus or baculovirus, so the production of said VLPs is carried out from mammal or insect cells; however, these production systems are very expensive for their application in the production at industrial level of veterinarian vaccines;
- ii): the production of VLPs of IBDV in mammal cells is based on the use of recombinants of the vaccine virus; however, in addition to the high cost of this production system, the use of a recombinant virus capable of infecting both mammas and birds does not meet the biosafety conditions necessary for its use as a vaccine;
- iii): in addition to the high cost of the production of VLPs of IBDV in insect cells using conventional expression systems, for example rBVs only expressing the viral polyprotein, it is very inefficient and leads to a virtually nule production of VLPs; and
- iv): the production of VLPs of IBDV in insect cells by means of the expression of a chimeric polyprotein, formed by the fusion of the open reading frame (ORF) corresponding to a heterologous protein at the 3' end of the ORF corresponding to the IBDV polyprotein, results in the production of VLPs of IBDV containing a fusion protein (VP3-heterologous protein), which introduces a protein element not present in IBDV virions, with an unknown effect and doubtful applicability in the production chain of chicken meat for human consumption.

It is therefore necessary to develop a vector that allows producing at the industrial level vaccines against IBDV which corrects all or part of the drawbacks hereinbefore discussed.

Yeasts are an alternative to the hereinbefore discussed expression systems due to simplicity and production costs. However, until now, the possibility of producing capsids in yeasts formed by the assembly of the VP2 protein of IBDV expressed in yeasts has not been described. In contrast, the results obtained by several groups of investigators point in the opposite direction, that is, the expression of the VP2 protein of IBDV in yeasts does not lead to the formation of particles or capsids. In relation thereto, Azad et al. (US 5,614,409) disclose the production of a highly immunogenic form of a recombinant VP2 (rVP2) protein of IBDV, wherein the last 5 amino acid residues of the amino terminal end of the native VP2 of IBDV have been replaced by an octapeptide by means of the expression of the encoding sequence of said rVP2 in yeasts. However, as the same authors acknowledge, electron microscopy analysis of the obtained material did not reveal the formation of defined particle structures (US 5,614,409, column 8, lines 64-67). Similar results seem to have been obtained by Pitcovski et al. ( Pitcovski J et al. 2003. Development and large-scale use of recombinant VP2 vaccine for the prevention of infectious bursal disease of chickens. Vaccine 21:4736-4743) who, although they disclose the expression of the gene encoding for the VP2 protein of IBDV in *Pichia pastoris* and the use of a partially purified material containing said rVP2 protein of IBDV for immunizing animals, do not disclose the formation of capsids by self-assembly of said rVP2 expressed in *P. pastoris.*

### SUMMARY OF THE INVENTION

Now it has surprisingly been found that it is possible to obtain empty capsids of IBDV by means of the expression and assembly of a pVP2* protein of IBDV, where said pVP2* protein is a protein the amino acid sequence of which consists of the amino acid sequence comprised between residue 1 and residue "n" of the pVP2 protein of IBDV, where "n" is an integer comprised between 441 and 501, in yeasts. Said empty capsids of IBDV, formed by self-assembly of said pVP2* protein of IBDV, are referred to as VLPs-pVP2* in this description. Said VLPs-pVP2* of IBDV contain one of the most antigenically relevant protein elements present in infective virions of IBDV, so they are able to induce an immune or antigenic response in an animal, and can therefore be used for therapeutic or diagnostic purposes.

It has specifically been observed that the expression of a pVP2* protein of IBDV in *Saccharomyces cerevisiae* allows obtaining VLPs-pVP2* of IBDV. Said VLPs-pVP2* have isometry T=1 and are immunogenic.

These results have allowed designing a new strategy for producing the VLPs of IBDV provided by this invention (VLPs-pVP2*) which, unlike the methods previously described for producing VLPs of IBDV (generally based on the use of recombinant viruses derived from the vaccine virus or from a baculovirus and in producing said VLPs from mammal or insect cells, unacceptable production systems for the industrial production of veterinarian vaccines for biosafety, efficiency and cost reasons), allows expressing and producing VLPs of IBDV in yeasts thereby correcting the problems of biosafety and efficiency, and a significant reduction of production costs, which allows its use in the industrial production of vaccines against IBDV. Said strategy is based on the use of a gene expression system or vector which allows expressing a pVP2* of IBDV in yeasts and forming VLPs-pVP2* of IBDV, with a very high yield and very low economic cost.

The vaccines obtained by using said VLPs-pVP2* have several advantages since, on one hand, the handling of highly infectious material is avoided, the potential risk of the occurrence of new IBDV mutants is prevented and the use of a live virus in poultry farms is removed, thus preventing the risk of spreading vaccine strains of IBDV into the environment, and on the other hand, it allows the development of differential diagnostic systems to discriminate between vaccinated and infected animals. These differential diagnostic systems are based on detecting antibodies against VP2, VP3 and VP4 proteins of IBDV. Animals with IBDV develop a strong humoral response to both proteins, whereas animals immunized with VLPs-pVP2* only have antibodies against the VP2 protein of IBDV.

Therefore, in one aspect, the present invention relates to a process for producing said VLPs-pVP2* of IBDV based on the gene expression of a pVP2* protein of IBDV in yeasts.

The expression systems and yeasts developed for putting said process of producing said VLPs-pVP2* of IBDV into practice, as well as their use for producing said VLPs-pVP2* of IBDV, constitute additional aspects of the present invention.

Said VLPs-pVP2* of IBDV, which have antigenic or immunogenic activity against the infection caused by IBDV, constitute an additional aspect of this invention. Likewise, the use of said VLPs-pVP2* of IBDV in preparing medicinal products, such as gene therapy vaccines and vectors, and/or for diagnostic purposes, constitutes an additional aspect of this invention. Said vaccines and vectors constitute additional aspects of the present invention.

### BRIEF DESCRIPTION OF THE FIGURES

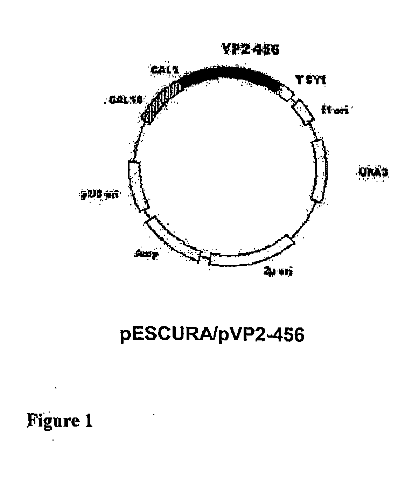

Figure 1 shows a schematic representation of the plasmid pESCURAinv/pVP2-456. The plasmid contains a DNA fragment containing the region corresponding to the polyprotein of IBDV which encodes from residue 1 to residue 456 of the VP2 protein of IBDV, under the transcriptional control of the promoter inducible by galactose called GAL1. This plasmid was used to transform a culture of the *Saccharomyces cerevisiae* haploid yeast strain 499.

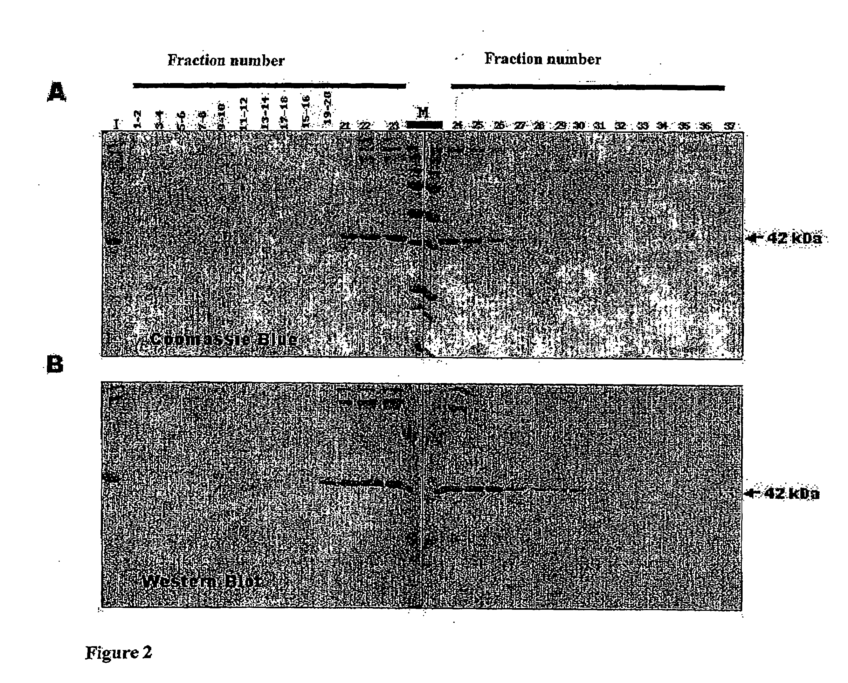

Figure 2 shows the purification of structures derived from IBDV in *S. cerevisiae* cells transformed with pESCURAinv/pVP2-456 and grown in the presence of galactose (inducer) for 18 hours at 30°C. The cultures were harvested and used to obtain extracts that were subsequently analyzed by means of ultracentrifugation in linear sucrose gradients. The lanes include samples corresponding to the total extract without fractionating (I) and to the different fractions from the lower area to the upper area of the gradient (1-37) by means of SDS-PAGE and staining with Coomassie blue (Figure 2A), and by means of Western blot using anti-VP2 serum of IBDV (Figure 2B). M indicates the lanes loaded with molecular weight markers (Dual Color, BIO-RAD).

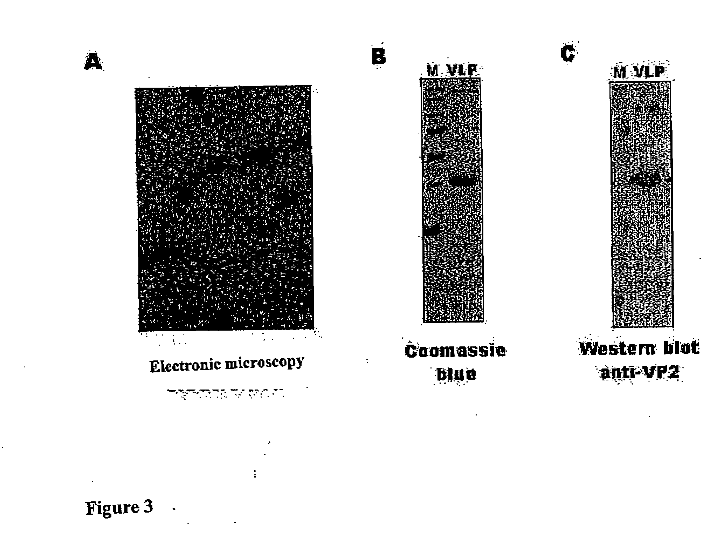

Figure 3 shows the structural characterization of assemblies derived from IBDV produced in *S. cerevisiae* cells transformed with pESCURAinv/pVP2-456. Figure 3A shows the microscopic characterization of purified VLPs-pVP2*; fractions 21 to 26 of the gradient shown in Figure 2 were mixed and analyzed by means of transmission electron microscopy (TEM); the presence of numerous isometric particles with a size of about 23 nm can be observed in the image. The sample was stained with uranyl acetate. Figure 3B shows the result of a biochemical analysis, specifically the sample (VLP) was analyzed by means of SDS-PAGE followed by staining with Coomassie blue. The molecular weight marker (M) used was Dual Color (BIO-RAD). Figure 3C shows the result of an antigenic analysis, specifically the sample (VLP) was analyzed by means of Western blot using anti-VP2 serum of IBDV. In Figures 3B and 3C, "M" indicates the lane corresponding to the molecular weight markers (Dual Color, BIO-RAD).

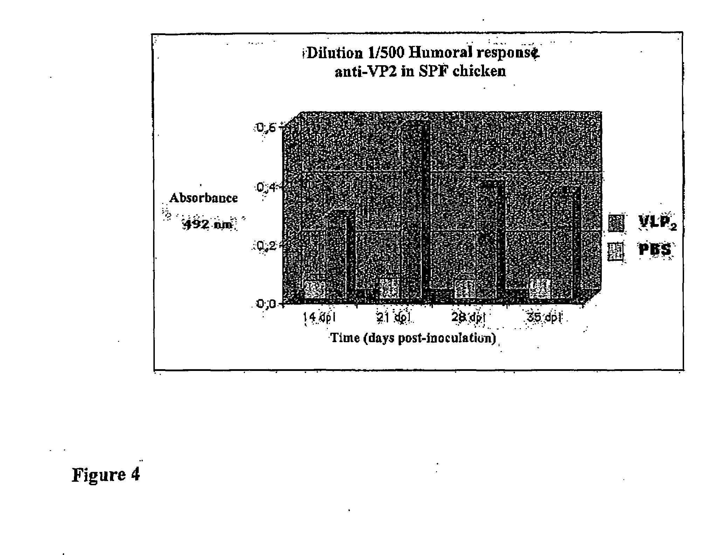

Figure 4 shows a bar graph illustrating the characterization of the humoral response to VLPs-pVP2-456. Said VLPs-pVP2-456 were analyzed to immunize 1-day old SPF chickens (Example 2). Immunization was carried out by using a group of 7 chickens with a single antigen dose administered intramuscularly. A group of chickens that were injected intramuscularly with an identical volume of the antigen diluent (PBS) was used as a control. Samples were taken at 7-day intervals until day 35. The serum samples were analyzed by means of ELISA using a 1/500 dilution of the serum sample.

### DETAILED DESCRIPTION OF THE INVENTION

### Definitions

As it is used in this description, the term VLP-pVP2* (singular) or VLPs-pVP2* (plural) of IBDV refers to empty capsids of the infectious bursal disease virus (IBDV), occasionally generically referred to as VLP-pVP2* or VLPs-pVP2* of the invention, with isometry T=1, formed by assembly of a pVP2* protein, where said pVP2* protein is a protein the amino acid sequence of which consists of the amino acid sequence comprised between residue 1 and residue "n" of the pVP2 protein of IBDV, where "n" is an integer comprised between 441 and 501.

As it is used in the present invention the term "IBDV" refers to the different IBDV strains belonging to any of the known serotypes (1 or 2) [by way of illustration, see the review carried out by van den Berg TP, Eterradossi N, Toquin D, Meulemans G., in Rev Sci Tech 2000 19: 509-43].

The term "pVP2* protein" generally refers to a protein the amino acid sequence of which consists of the amino acid sequence comprised between residue 1 and residue "n" of the pVP2 protein of IBDV, where "n" is an integer comprised between 441 and 501 and includes any of the different forms of the pVP2 proteins representative of any of the mentioned strains of IBDV [NCBI protein databank], according to the definition given by Sánchez and Rodriguez (1999) (Sánchez AB, Rodríguez JF. Proteolytic processing in infectious bursal disease virus: identification of the polyprotein cleavage sites by site-directed mutagenesis. Virology. 1999 Scp 15; 262(1):190-199), as well as to proteins substantially homologous to said pVP2 proteins of IBDV, i.e. proteins the amino acid sequences of which have a degree of identity with respect to said pVP2 proteins of IBDV of at least 60%, preferably of at least 80%, more preferably of at least 90% and, even more preferably of at least 95%.

The particular pVP2* proteins are referred to by following the format "pVP2-n", where "n" is as hereinbefore defined. In a particular embodiment, said pVP2* protein is a protein selected from the group formed by:
- (i): the pVP2 protein-441, the amino acid sequence of which consists of the amino acid sequence comprised between residue 1 and residue 441 of the pVP2 protein of IBDV;
- (ii): the pVP2 protein-452, the amino acid sequence of which consists of the amino acid sequence comprised between residue 1 and residue 452 of the pVP2 protein of IBDV;
- (iii): the pVP2-456 protein, the amino acid sequence of which consists of the amino acid sequence comprised between residue I and residue 456 of the pVP2 protein of IBDV;
- (iv): the pVP2 protein-466, the amino acid sequence of which consists of the amino acid sequence comprised between residue 1 and residue 466 of the pVP2 protein of IBDV;
- (v): the pVP2 protein-476, the amino acid sequence of which consists of the amino acid sequence comprised between residue 1 and residue 476 of the pVP2 protein of IBDV;
- (vi): the pVP2 protein-487, the amino acid sequence of which consists of the amino acid sequence comprised between residue 1 and residue 487 of the pVP2 protein of IBDV;
- (vii): the pVP2 protein-494, the amino acid sequence of which consists of the amino acid sequence comprised between residue 1 and residue 494 of the pV P2 protein of IBDV; and
- (viii): the pVP2 protein-501, the amino acid sequence of which consists of the amino acid sequence comprised between residue 1 and residue 501 of the pVP2 protein of IBDV.

The pVP2* protein of IBDV present in the VLPs-pVP2* provided by this invention has an amino acid sequence comprising between 441 and 501 amino acid residues, starting from residue 1, of any pVP2 protein representative of any IBDV strain, for example, the pV.P2 protein of the IBDV Soroa strain [NCBI, access number AAD30136]. In a particular embodiment, said pVP2* protein of IBDV present in the VLPs-pVP2* provided by this invention is the pVP2-456 protein the amino acid sequence of which is comprised between residue 1 and residue 456 of the pVP2 protein of IBDV Soroa strain [NCBI, access number AAD30136]. The nucleotide sequence encoding for said pVP2-456 protein of IBDV extends from nucleotide 350 to nucleotide 1719 of the nucleotide sequence shown in SEQ. ID. NO: 1.

As it is used in this description, the term "open reading frame corresponding to the pVP2* protein of IBDV" includes, in addition to the nucleotide sequences of said open reading frames, other open reading frames analogous to the same ones encoding pVP2* proteins of IBDV.

As it is herein used, the term "analogous" aims to include any nucleotide sequence that can be isolated or constructed on the basis of the encoding nucleotide sequences of pVP2* of IBDV, for example by means of introducing conservative or non-conservative nucleotide substitutions, including the insertion of one or more nucleotides, the addition of one or more nucleotides at any of the ends of the molecule or the deletion of one or more nucleotides at any end or within the sequence. Generally, a nucleotide sequence analogous to another nucleotide sequence is substantially homologous to said nucleotide sequence. In the sense used in this description, the expression "substantially homologous" means that the nucleotide sequences involved have a degree of identity, at the level of nucleotides, of at least 60%, preferably of at least 80%, more preferably of at least 90% and even more preferably of at least 95%.

### VLPs-pVP2* of IBDV: process for producing them and applications

The VLPs-pVP2* provided by this invention can be obtained by means of the expression of said pVP2* protein of IBDV, in suitable host cells, particularly yeasts, containing the nucleotide sequence encoding for said pVP2* protein of IBDV in a gene construct. In a particular embodiment, said suitable host cells are yeasts transformed with a suitable expression system including a gene construct comprising the nucleotide sequence encoding for said pVP2* protein of IBDV.

In one aspect, the invention relates to a process for producing VLPs-pVP2* of IBDV, hereinafter process of the invention, which comprises culturing a yeast containing the nucleotide sequence encoding for a pVP2* protein of IBDV and expressing said pVP2* protein of IBDV, and if desired, recovering said VLPs-pVP2* of the invention.

In a particular embodiment, the process of the invention comprises the steps of:
a) culturing yeast cells transformed with an expression system comprising the nucleotide sequence encoding for a pVP2* protein of IBDV, wherein said pVP2* protein is a protein the amino acid sequence of which consists of the amino acid sequence comprised between residue 1 and residue "n" of the pVP2 protein of IBDV, where "n" is an integer comprised between 441 and 501, under conditions allowing the expression of said pVP2* proteins and their assembly for forming VLPs-pVP2* of IBDV; and
b) if desired, isolating and optionally purifying said VLPs-pVP2* of IBDV.

As can be seen, the process of the invention comprises the gene expression of a pVP2* protein of IBDV by means of the use of a vector allowing the expression of said protein in yeasts. To that end, the process of the invention comprises, as a prior step, obtaining a gene expression system, such as a system consisting of a plasmid containing a gene construct encoding for said pVP2* protein of IBDV, followed by the transformation of yeasts with said expression system, the expression of the recombinant proteins, and if desired the isolation of the VLPs-pVP2* of IBDV formed by assembly of said pVP2* proteins of IBDV, and optionally the purification of said VLPs-pVP2*.

Obtaining yeasts transformed with an expression system or vector allowing the expression of the pVP2* protein of IBDV can be carried out by a person skilled in the art based on that herein described and on the state of the art on this technology (pESC epitope tagging vectors Instructions manual. Stratagene www.stratagene.com; Sambrook J.1989. Molecular cloning: a laboratory manual, 2nd ed. Cold Spring Harbor Laboratory, Cold Spring Harbor, N.Y.). The transformed yeasts are cultured under conditions, known by persons skilled in the art, allowing the expression of recombinant proteins (pVP2*) and their assembly to form VLPs-pVP2* of IBDV. After the expression of said pVP2* protein of TBDV in yeasts, the expressed pVP2* proteins are assembled and form VLPs-pVP2* of IBDV, which can be isolated or removed from the medium and purified if desired. The isolation and purification of said VLPs-pVP2* of IBDV of the invention can be carried out by conventional methods, for example by means of fractionating in sucrose gradients.

The expression system or vector used to transform yeasts comprises the nucleotide sequence encoding for a pVP2* protein of IBDV operatively bound to transcription, and optionally translation, control elements and constitutes an additional aspect of this invention.

Therefore, in another aspect, the invention provides an expression system or vector useful for transforming yeasts, comprising a nucleotide sequence encoding for a pVP2* protein of IBDV, where said pVP2* protein is a protein the amino acid sequence of which consists of the amino acid sequence comprised between residue 1 and residue "n" of the pVP2 protein of IBDV, where "n" is an integer comprised between 441 and 501, said nucleotide sequence encoding for said pVP2* protein of IBDV operatively bound to transcription, and optionally translation, control elements.

In a particular embodiment, said expression system provided by this invention comprises the nucleotide sequence comprising the open reading frame or encoding region corresponding to a pVP2* protein selected from the pVP2-441, pVP2-452, pVP2-456, pVP2-466, pVP2-476, pVP2-487, pVP2-494 and pVP2-501 proteins.

The transcription, and optionally translation, control elements, present in said expression system include promoters which direct the transcription of the sequence of the pVP2* protein (to which it is operatively bound), and other sequences required or suitable for transcription and their suitable regulation in time and place, for example initiation and termination signals, cutting sites, polyadenylation signal, replication origin, transcriptional enhancers, transcriptional silencers, etc., all being useful in yeasts.

Virtually any suitable expression system or vector can be used in generating said expression system. By way of illustration, said vectors include plasmids, yeast artificial chromosomes (YACs), etc., that may further contain a heterologous replication origin, for example bacterial, so that it can be amplified in bacteria, as well as a marker that can be used to select the transfected cells that is different from the gene or genes of interest. These expression vectors can be obtained by conventional methods known by persons skilled in the art [Sambrook J et al. 1989. Molecular cloning: a laboratory manual, 2nd ed. Cold Spring Harbor Laboratory] and form part of the present invention. In a particular embodiment, said expression system or vector is a plasmid, such as a plasmid suitable for transforming yeasts based on a pESC Yeast (Stratagene) expression system, for example the plasmid pESCURA/pVP2-456 (Example 1.1) which contains a gene construct encoding for the pVP2-456 protein of IBDV and allows producing VLPs-pVP2* of LBDV in yeasts.

The use of the gene expression system provided by this invention for producing and obtaining VLPs-pVP2* of IBDV constitutes an additional aspect of this invention.

In another aspect, the invention provides a host cell, such as a yeast, containing a nucleotide sequence encoding for said pVP2* protein of IBDV. In a particular embodiment, said host cell is a yeast transformed with an expression system provided by this invention comprising a gene construct comprising the nucleotide sequence encoding for said pVP2* protein of IBDV. Virtually any yeast can be used for putting the process of the invention into practice; nevertheless, in a particular embodiment, said yeast is a yeast of the *Saccharomyces* genus, for example, *S*. *cerevisae, S. pombe,* etc., a yeast of the *Pichia* genus, for example, *P. pastoris,* etc.

The VLPs-pVP2* of IBDV can be used as vectors or carriers of products of interest, such as molecules with biological activity, for example, drugs, polypeptides, proteins, nucleic acids, etc., so they can be used for therapeutic, diagnostic or investigational purposes. In a particular embodiment, said molecules of biological interest include polypeptides of interest, such as antigens or inducers of immune responses in animals or humans they are administered to, or they include nucleic acid sequences, useful in gene therapy, intended to be introduced inside the suitable cells.

Said VLPs-pVP2* of IBDV can be used to immunize animals, particularly birds, *per se* or as vectors or carriers of molecules with biological activity, for example, polypeptides, proteins, nucleic acids, drugs, etc., so they can be used for therapeutic or diagnostic purposes. In a particular embodiment, said molecules with biological activity include antigens or inducers of immune responses in animals or humans they are administered to, or drugs released in their specific action site, or nucleic acid sequences, all of them being useful in gene therapy and intended to be introduced inside the suitable cells.

Therefore, in another aspect, the invention relates to the use of said VLPs-pVP2* of IBDV in preparing medicinal products such as, vaccines, gene therapy vectors (delivery systems), etc. In a particular embodiment, said medicinal product is a vaccine intended to provide protection to animals, particularly birds, against IBDV. In another particular embodiment, said medicinal product is a gene therapy vector.

In another aspect, the invention provides a vaccine comprising a therapeutically effective amount of VLPs-pVP2* of IBDV, optionally with one or more pharmaceutically acceptable adjuvants and/or carriers. Said vaccine is useful for protecting animals, particularly birds, against IBDV. In a particular embodiment, said birds are selected from the group formed by chickens, turkeys, geese, gander, pheasants, quails and ostriches. In a preferred embodiment, the vaccine provided by this invention is a vaccine useful for protecting chickens from the infection caused by IBDV.

In the sense used in this description, the expression "therapeutically effective amount" refers to the amount of VLPs-pVP2* of the invention calculated to produce the desired effect, and will generally be determined, among other causes, by the characteristics of the VLPs-pVP2* of the invention and the immunization effect to be obtained.

The adjuvants and pharmaceutically acceptable carriers that can be used in said vaccines are the adjuvants and carriers known by persons skilled in the art and habitually used in preparing vaccines.

Jn a particular embodiment, said vaccine is prepared in the form of a solution or aqueous suspension, in a pharmaceutically acceptable diluent, such as saline solution, phosphate buffered saline solution (PBS), or any other pharmaceutically acceptable diluent.

The vaccine provided by this invention can be administered by any suitable administration route which results in a protective immune response against the heterologous sequence or epitope used, for which purpose said vaccine will be formulated in the pharmaceutical form that is suitable for the chosen administration route. In a particular embodiment, administration of the vaccine provided by this invention is carried out parenterally, for example, intraperitoneally, subcutaneously, etc.

The following Examples illustrate the invention and should not be considered to be limiting of the scope thereof. Example 1 illustrates obtaining VLPs-pVP2-456 of IBDV, whereas Example 2 shows the antigenic capacity of said VLPs-pVP2-456 of IBDV in chickens.

### EXAMPLE 1

### Obtaining VLPs-pVP2* by means of the expression of various regions of the pVP2 protein in yeasts

### 1.1 Obtaining VLPs-pVP2-465 by means of the expression of the 1-456 region of the pVP2 protein in yeasts

For the purpose of studying the possibility of obtaining VLPs of IBDV formed by self-assembly of the pVP2-456 protein, the amino acid sequence of which consists of the amino acid sequence comprised between residue 1 and residue 456 of the pVP2 protein of IBDV, called VLPs-pVP2-456, in yeast cultures (*Saccharomyces cerevisiae),* the vector pESCURAinv/pVP2-456 was generated. The first step in the construction of the vector was carried out by means of cloning the encoding region of the pVP2-456 protein (residues 1-456 of the pVP2 of IBDV) in the vector pESCURAinv. The plasmid pESCURAinv was generated by means of digesting the vector pRS426 (Stratagene) with the enzyme PvuII and religating the digestion mixture. The resulting vector, pESCURAinv, contains the multiple cloning region in reverse position with respect to that of the parent vector pRS426. The DNA fragment corresponding to the pVP2-456 protein was obtained by means of PCR with the oligonucleotides identified as Oligo I (SEQ ID NO: 2) and Oligo II (SEQ ID NO: 3) using the pVOTE.2/Poly plasmid as a mold (Fernández-Arias A et al. 1998. Expression of ORF A1 of infectious bursal disease virus infectious bursal disease virus results in the formation of virus-like particles. Journal of General Virology 79:1047-1054). The fragment was purified, subjected to digestion with the enzymes BgIII and HindIII and cloned into the vector pESCURA.inv previously digested with the enzymes BamHI and HindIII. The resulting plasmid was called pESCURAinv/pVP2-456 (Figure 1).

The plasmid pESCURAinv/pVP2-456 was used to transform a culture of the S. *cerevisiae* yeast haploid strain 499 according to a previously disclosed protocol (Gietz & Woods. 2002. Transformation of yeast by the Liac/SS carrier DNA/PEG method. Methods in Enzymology 350:87-96). The yeasts transformed with the plasmid were selected by means of growth in dishes with SC medium (CSM + YNB, 2% glucose and bacto agar) supplemented with the amino acids tryptophan, leucine and histidine and lacking uracyl (-Ura). After 48 hours of incubation at 30°C, a colony was selected that was used to conduct the subsequent analyses of protein expression and the formation of VLPs. The analysis of the expression of the pVP2-456 protein and the formation of VLPs was carried out following a protocol previously described for the characterization of VLPs of IBDV in other expression systems (Fernandez-Arias A et al. 1998. Expression of ORF A1 of infectious bursal disease virus results in the formation of virus-like particles. Journal of General Virology 79, 1047-1054; Lombardo E ct al. 1999. VP1, the putative RNA-dependent RNA polymerase of infectious bursal disease virus, terms complexes with the capsid protein VP3, leading to efficient encapsidation into virus-like particles. Journal of Virology 73:6973-698).

The selected colony was cultured in CSM (-Ura) + YNB liquid medium supplemented with 2% raffinose. The culture was incubated at 30°C for 24 hours. This culture was used to inoculate, at an optical density (OD) of 0.2, a 200 ml flask of CSM (-Ura) + YNB medium supplemented with the 2% galactose inducer. The culture was maintained at 30°C for 18 hours (up to an OD between 1.0 and 2.0). The yeasts were centrifuged at 3,000 rpm, 5 minutes at 4°C, were washed with distilled water once, and the pellet was resuspended in lysis buffer (TEN: 10 mM Tris, pH 8.0; 150 mM NaCl; 1 mM EDTA) + protease inhibitors 2X (Compl Roche). One volume of glass beads with an approximate size of 425-600 microns (Sigma) was added for lysis. This mixture was subjected to a vigorous vortex for 30 seconds 4 times, with 30 second intervals, and all at 4°C. Then the soluble fraction was recovered by centrifuging the lysis mixture at 13,000 rpm for 15 minutes at 4°C. This sample was subjected to fractionation in a sucrose gradient according to the previously disclosed protocol (Lombardo E ct al. 1999. VP1, the putative RNA-dependent RNA polymerase of infectious bursal disease virus, forms complexes with the capsid protein VP3, leading to efficient encapsidation into virus-like particles. Journal of Virology 73:6973-6983). The samples obtained after fractionation and a sample of the starting material were analyzed by means of sodium dodecyl sulfate polyacrylamide gel electrophoresis (SDS-PAGE) [Current Protocols in Molecular Biology] [Current Protocols in Molecular Biology, Edited by: Fred M. Ausubel, Roger Brent, Robert E. Kingston, David D. Moore, J.G. Seidman, John A. Smith, Kevin Struhl, John Wiley & Sons, http://www.interscience.wiley.com/c_p/index.htm] and staining with Coomassie blue and immunodetection by Western blot using anti-pVP2 serum [Current Protocols in Molecular Biology, Edited by: Fred M. Ausubel, Roger Brent, Robert E. Kingston, David D. Moore, J.G. Seidman, John A. Smith, Kevin Struhl, John Wiley & Sons, http://www.interscience.wiley.com/c_p/index.htm]. The obtained results are shown in Figure 2. As can be seen in Figure 2A, staining with Coomassie blue revealed the presence of a protein with the size (42 kDa) expected for pVP2-456, together with proteins with a greater molecular mass, in different fractions corresponding to the central area of the gradient. Figure 2B, corresponding to Western blot analysis, shows that the bands detected by means of staining with Coomassie blue correspond to the pVP2-456 protein both in its monomeric form and in the form of high molecular weight oligomers. These results irrefutably showed the correct expression of the polypeptide pVP2-456 in the *S. cerevisiae* culture transformed with the plasmid pESCURAinv/pVP2-456.

Then, the various fractions of the gradient were analyzed by means of transmission electron microscopy as previously described (Lombardo E et al. 1999. VP1, the putative RNA-dependent RNA polymerase of infectious bursal disease virus, forms complexes with the capsid protein VP3, leading to efficient encapsidation into virus-like particles. Journal of Virology 73:6973-6983). This analysis was carried out with the product resulting from mixing fractions 21 to 26 of the gradient indicated in Figure 2. As shown in Figure 3A, the TEM analysis of said fractions of the gradient revealed the existence of VLPs of IBDV in that region of the gradient. The VLPs obtained (VLPs-pVP2-456) arc isometrie (T = 1) and have a diameter of 23 nm and a circular appearance. The subsequent SDS-PAGE analysis (Figure 3B) and immunoblotting analysis with anti-VP2 scrum (Figure 3C), showed that the fraction contained the pVP2-456 protein with a degree of purity estimated to be at least 95%.

### 1.2 Obtaining VLPs-pVP2* by means of the expression of other regions of the pVP2 protein in yeasts

Following the process described in Example 1.1, the vectors described in Table 1 were generated, said vectors expressed the VP2* in yeasts listed in said Table 1, which further includes the isometry of the VLPs-pVP2* obtained and the primers used.

**Table 1**

| **Plasmid** | **pVP2*** | **Isometry** | **Forward primer** | **Reverse primer** |
|---|---|---|---|---|
| pESCURAinv/pVP2-441 | pVP2-441 | (a) | SEQ. ID. NO: 2 | SEQ. ID. NO: 4 |
| pESCURAinv/pVP2-452 | pVP2-452 | (a) | SPQ. ID. NO: 2 | SEQ. ID. NO: 5 |
| pESCURAinv/pVP2-466 | pVP2-466 | (a) (b) (c) (d) | SEQ. ID. NO: 2 | SEQ. ID. NO: 6 |
| pESCURAinv/pVP2-476 | pVP2-476 | (a) (b) (e) (f) | SEQ. ID. NO: 2 | SEQ. ID. NO: 7 |
| pESCURAinv/pVP2-487 | pVP2-487 | N.D. | SEQ. ID. NO: 2 | SEQ. ID. NO: 8 |
| pESCURAinv/pVP2-494 | pVP2-494 | N.D. | SEQ. ID. NO: 2 | SEQ. ID. NO: 9 |
| pESCURAinv/pVP2-501 | pVP2-501 | N.D. | SEQ. ID. NO: 2 | SEQ. ID. NO: 10 |
| pESCURAinv/pVP2-512 | pVP2-512 | (f) | SEQ. ID. NO: 2 | SEQ. ID. NO: 11 |

| | | | | |
|---|---|---|---|---|
| NOTES: * The number indicates the number of amino acid residues of pVP2 of IBDV of each protein. (a) VLPs T = 1 (b) VLPs T = 7 (c) 20 nm tubules (d) Capsomers (e) VLPs T =13 (f) 50 nm tubules | | | | |

### EXAMPLE 2

### Characterization of the immunogenicity of the VLPs-pVP2-456 of IBDV

An immunization test was conducted in 1-day old chickens for the purpose of determining the immunogenicity of the VLPs-pVP2-456 (Example 1.1). A group of 7 SPF (specific pathogen free) animals was intramuscularly immunized with a single dose of 200 µl containing 10 µg of VLPs-pVP2-456/animal diluted in PBS. A similar group was injected with PBS. Weekly serum extractions from each one of the animals of both groups were performed. The serums of each group and date were mixed to obtain a homogenous serum (pool) represented by equivalent volumes of each individual of the group. The serums were analyzed by means of ELISA. To that end, the wells were coated with 10 ng of VLPs-pVP2-456. The tests were conducted according to a previously described protocol (Current Protocols in Immunology. Edited by: Barbara Bierer, John E. Coligan, David H. Margulies, Ethan M.Shevach, Warren Strober, John Wiley & Sons http://www.interscience.wiley.com/c_p/index.htm). The obtained results, reflected in Figure 4, show that a single immunization in the absence of an adjuvant causes a potent response to the pVP2-456 protein.

## Claims

1. A process for producing empty capsids of the infectious bursal disease virus (IBDV) [VLPs-pVP2*] which comprises culturing a yeast containing the nucleotide sequence encoding for a pVP2* protein of IBDV and expressing said pVP2* protein of IBDV, and if desired recovering said VLPs-pVP2*, where said pVP2* protein of IBDV is a protein the amino acid sequence of which consists of the amino acid sequence comprised between residue 1 and residue "n" of the pVP2 protein of IBDV, where "n" is an integer comprised between 441 and 501.

2. A process according to claim 1, comprising the steps of:
a) culturing yeast cells transformed with an expression system comprising the nucleotide sequence encoding for a pVP2* protein of IBDV, under conditions allowing the expression of said pVP2* proteins and their assembly for forming VLPs-pVP2* of IBDV; and
b) if desired, isolating and optionally purifying said VLPs-pVP2* of IBDV.

3. A process according to any of claims 1 or 2, wherein said pVP2* protein of IBDV is selected from the group formed by:
(i) the pVP2 protein-441, the amino acid sequence of which consists of the amino acid sequence comprised between residue 1 and residue 441 of the pVP2 protein of IBDV;
(ii) the pVP2 protein-452, the amino acid sequence of which consists of the amino acid sequence comprised between residue 1 and residue 452 of the pVP2 protein of IBDV;
(iii) the pVP2-456 protein, the amino acid sequence of which consists of the amino acid sequence comprised between residue 1 and residue 456 of the pVP2 protein of IBDV;
(iv) the pVP2 protein-466, the amino acid sequence of which consists of the amino acid sequence comprised between residue 1 and residue 466 of the pVP2 protein of IBDV;
(v) the pVP2 protein-476, the amino acid sequence of which consists of the amino acid sequence comprised between residue 1 and residue 476 of the pVP2 protein of IBDV;
(vi) the pVP2 protein-487, the amino acid sequence of which consists of the amino acid sequence comprised between residue 1 and residue 487 of the pVP2 protein of IBDV;
(vii) the pVP2 protcin-494, the amino acid sequence of which consists of the amino acid sequence comprised between residue 1 and residue 494 of the pVP2 protein of IBDV; and
(viii) the pVP2 protein-501, the amino acid sequence of which consists of the amino acid sequence comprised between residue 1 and residue 501 of the pVP2 protein of IBDV.

4. A process according to any of claims 1 to 3, wherein said yeast is of the *Saccharomyces* genus or the *Pichia* genus.

5. A process according to claim 4, wherein said yeast is *S. cerevisiae, S. pombe* or *P. pastoris.*

6. An expression system useful for transforming yeasts, comprising the nucleotide sequence encoding for a pVP2* protein of IBDV operatively bound to transcription, and optionally translation, control elements, where said pVP2* protein is a protein the amino acid sequence of which consists of the amino acid sequence comprised between residue 1 and residue "n" of the pVP2 protein of IBDV, where "n" is an integer comprised between 441 and 501.

7. A yeast comprising an expression system according to claim 6.

8. A yeast transformed with an expression system according to claim 6.

9. A yeast according to any of claims 7 or 8, wherein said yeast is of the *Saccharomyces* genus.

10. A yeast according to claim 9, wherein said yeast is S. *cerevisiae* or *S. pombe.*

11. Use of an expression system according to claim 6, or of a yeast according to any of claims 7 to 10, for producing empty viral capsids of the infectious bursal disease virus (IBDV) [VLPs-pVP2*].

12. An empty capsid of the infectious bursal disease virus (IBDV) [VLP-pVP2*] obtained according to the process of any of claims 1 to 5.

13. An empty capsid of the infectious bursal disease virus (IBDV) [VLP-pVP2*], **characterized in that** it is formed by assembly of pVP2* proteins of IBDV expressed in yeasts, where said pVP2* protein of IBDV is a protein the amino acid sequence of which consists of the amino acid sequence comprised between residue 1 and residue "n" of the pVP2 protein of IBDV, where "n" is an integer comprised between 441 and 501.

14. A capsid according to any of claims 12 or 13, wherein said pVP2* protein of IBDV is selected from the group formed by:
(i) the pVP2 protein-441, the amino acid sequence of which consists of the amino acid sequence comprised between residue 1 and residue 441 of the pVP2 protein of IBDV;
(ii) the pVP2 protein-452, the amino acid sequence of which consists of the amino acid sequence comprised between residue 1 and residue 452 of the pVP2 protein of IBDV;
(iii) the pVP2-456 protein, the amino acid sequence of which consists of the amino acid sequence comprised between residue 1 and residue 456 of the pVP2 protein of IBDV;
(iv) the pVP2 protein-466, the amino acid sequence of which consists of the amino acid sequence comprised between residue 1 and residue 466 of the pVP2 protein of IBDV;
(v) the pVP2 protein-476, the amino acid sequence of which consists of the amino acid sequence comprised between residue 1 and residue 476 of the pVP2 protein of IBDV;
(vi) the pVP2 protein-487, the amino acid sequence of which consists of the amino acid sequence comprised between residue 1 and residue 487 of the pVP2 protein oC IBDV;
(vii) the pVP2 protein-494, the amino acid sequence of which consists of the amino acid sequence comprised between residue 1 and residue 494 of the pVP2 protein of IBDV; and
(viii) the pVP2 protein-501, the amino acid sequence of which consists of the amino acid sequence comprised between residue 1 and residue 501 of the pVP2 protein of IBDV.

15. A capsid according to any of claims 12 to 14, **characterised in that** it has isometry T=1.

16. The use of empty capsids of the infectious bursal disease virus (IBDV) [VLPs-pVP2*], according to any of claims 12 to 15, in preparing a medicinal product.

17. The use according to claim 16, wherein said medicinal product is a vaccine against the avian disease referred to as infectious bursitis, or a gene therapy vector.

18. A vaccine comprising a therapeutically effective amount of empty capsids of the infectious bursal disease virus (IBDV) [VLPs-pVP2*], according to any of claims 12 to 15, optionally with one or more pharmaceutically acceptable adjuvants and/or carriers.

19. A vaccine according to claim 18, for protecting birds from the infectious bursal disease virus (IBDV).

20. A vaccine according to claim 19, wherein said birds are selected from the group formed by chickens, turkeys, geese, gander, pheasants, quails and ostriches.

21. A vaccine for protecting chickens from the infectious bursal disease virus (IBDV) comprising a therapeutically effective amount of empty capsids of IBDV [VLPs-pVP2*], according to any of claims 12 to 15, optionally with one or more pharmaceutically acceptable adjuvants and/or carriers.


SEQUENCE LISTING

<110> CONSEJO SUPERIOR DE INVESTIGACIONES CIENTÍFICAS
<110> BIONOSTRA, S.L.

<120> PROCESS FOR PRODUCING IN YEASTS EMPTY VIRAL CAPSIDS CONSISTING OF
PROTEINS DERIVED FROM pVP2 OF THE INFECTIOUS BURSAL DISEASE VIRUS (IBDV)

<160> 11

<170> PatentIn version 3.1

<210> 1
<211> 7929
<212> DNA
<213> Artificial sequence

<220>
<223> Nucleotide sequence of plasmid pESCURAinv/pVP2-456

<220>
<221> promoter
<222> (1)..(342)
<223> Promoter Gal1

<220>
<221> CDS
<222> (350)..(1719)
<223> Open reading frame of IBDV pVP2-456 protein

<400> 1
cactgctccg aacaataaag attctacaat actagctttt atggttatga agaggaaaaa 60
ttggcagtaa cctggcccca caaaccttca aatgaacgaa tcaaattaac aaccatagga 120
tgataatgcg attagttttt tagccttatt tctggggtaa ttaatcagcg aagcgatgat 180
ttttgatcta ttaacagata tataaatgca aaaactgcat aaccacttta actaatactt 240
tcaacatttt cggtttgtat tacttcttat tcaaatgtaa taaaagtatc aacaaaaaat 300
tgttaatata cctctatact ttaacgtcaa ggagaaaaaa ccccggatct atgacaaacc 360
tgtcagatca aacccagcag attgttccgt tcatacggag ccttctgatg ccaacaaccg 420
gaccggcgtc cattccggac gacaccctgg agaagcacac tctcaggtca gagacctcga 480
cctacaattt gactgtgggg gacacagggt cagggctaat tgtctttttc cctggattcc 540
ctggctcaat tgtgggtgct cactacacac tgcagggcaa tgggaactac aagttcgatc 600
agatgctcct gactgcccag aacctaccgg ccagttacaa ctactgcagg ctagtgagtc 660
ggagtctcac agtgaggtca agcacacttc ctggtggcgt ttatgcacta aacggcacca 720
taaacgccgt gaccttccaa ggaagcctga gtgaactgac agatgttagc tacaatgggt 780
tgatgtctgc aacagccaac atcaacgaca aaattgggaa cgtcctagta ggggaagggg 840
tcaccgtcct cagcttaccc acatcatatg atcttgggta tgtgaggctt ggtgacccca 900
ttcccgcaat agggcttgac ccaaaaatgg tagccacatg tgacagcagt gacaggccca 960
gagtctacac cataactgca gccgatgatt accaattctc atcacagtac caaccaggtg 1020
gggtaacaat cacactgttc tcagccaaca ttgatgccat cacaagcctc agcgttgggg 1080
gagagctcgt gtttcgaaca agcgtccacg gccttgtact gggcgccacc atctacctca 1140
taggctttga tgggacaacg gtaatcacca gggctgtggc cgcaaacaat gggctgacga 1200
ccggcaccga caaccttatg ccattcaatc ttgtgattcc aacaaacgag ataacccagc 1260
caatcacatc catcaaactg gagatagtga cctccaaaag tggtggtcag gcaggggatc 1320
agatgtcatg gtcggcaaga gggagcctag cagtgacgat ccatggtggc aactatccag 1380
gggccctccg tcccgtcacg ctagtggcct acgaaagagt ggcaacagga tccgtcgtta 1440
cggtcgctgg ggtgagcaac ttcgagctga tcccaaatcc tgaactagca aagaacctgg 1500
ttacagaata cggccgattt gacccaggag ccatgaacta cacaaaattg atactgagtg 1560
agagggaccg tcttggcatc aagaccgtct ggccaacaag ggagtacact gactttcgtg 1620
aatacttcat ggaggtggcc gacctcaact ctcccctgaa gattgcagga gcattcggct 1680
tcaaagacat aatccgggcc ataaggagga tagctgtgta agcttggtac cgcggctagc 1740
taagatccgc tctaaccgaa aaggaaggag ttagacaacc tgaagtctag gtccctattt 1800

atttttttat agttatgtta gtattaagaa cgttatttat atttcaaatt tttctttttt 1860
ttctgtacag acgcgtgtac gcatgtaaca ttatactgaa aaccttgctt gagaaggttt 1920
tgggacgctc gaagatccag ctggcgtaat agcgaagagg cccgcaccga tcgcccttcc 1980
caacagttgc gcagcctgaa tggcgaatgg acgcgccctg tagcggcgca ttaagcgcgg 2040
cgggtgtggt ggttacgcgc agcgtgaccg ctacacttgc cagcgcccta gcgcccgctc 2100
ctttcgcttt cttcccttcc tttctcgcca cgttcgccgg ctttccccgt caagctctaa 2160
atcgggggct ccctttaggg ttccgattta gtgctttacg gcacctcgac cccaaaaaac 2220
ttgattaggg tgatggttca cgtagtgggc catcgccctg atagacggtt tttcgccctt 2280
tgacgttgga gtccacgttc tttaatagtg gactcttgtt ccaaactgga acaacactca 2340
accctatctc ggtctattct tttgatttat aagggatttt gccgatttcg gcctattggt 2400
taaaaaatga gctgatttaa caaaaattta acgcgaattt taacaaaata ttaacgttta 2460
caatttcctg atgcggtatt ttctccttac gcatctgtgc ggtatttcac accgcatagg 2520
gtaataactg atataattaa attgaagctc taatttgtga gtttagtata catgcattta 2580
cttataatac agttttttag ttttgctggc cgcatcttct caaatatgct tcccagcctg 2640
cttttctgta acgttcaccc tctaccttag catcccttcc ctttgcaaat agtcctcttc 2700
caacaataat aatgtcagat cctgtagaga ccacatcatc cacggttcta tactgttgac 2760
ccaatgcgtc tcccttgtca tctaaaccca caccgggtgt cataatcaac caatcgtaac 2820
cttcatctct tccacccatg tctctttgag caataaagcc gataacaaaa tctttgtcgc 2880
tcttcgcaat gtcaacagta cccttagtat attctccagt agatagggag cccttgcatg 2940
acaattctgc taacatcaaa aggcctctag gttcctttgt tacttcttct gccgcctgct 3000
tcaaaccgct aacaatacct gggcccacca caccgtgtgc attcgtaatg tctgcccatt 3060
ctgctattct gtatacaccc gcagagtact gcaatttgac tgtattacca atgtcagcaa 3120
attttctgtc ttcgaagagt aaaaaattgt acttggcgga taatgccttt agcggcttaa 3180
ctgtgccctc catggaaaaa tcagtcaaga tatccacatg tgtttttagt aaacaaattt 3240
tgggacctaa tgcttcaact aactccagta attccttggt ggtacgaaca tccaatgaag 3300
cacacaagtt tgtttgcttt tcgtgcatga tattaaatag cttggcagca acaggactag 3360
gatgagtagc agcacgttcc ttatatgtag ctttcgacat gatttatctt cgtttcctgc 3420
aggtttttgt tctgtgcagt tgggttaaga atactgggca atttcatgtt tcttcaacac 3480
tacatatgcg tatatatacc aatctaagtc tgtgctcctt ccttcgttct tccttctgtt 3540
cggagattac cgaatcaaaa aaatttcaaa gaaaccgaaa tcaaaaaaaa gaataaaaaa 3600
aaaatgatga attgaattga aaagctgtgg tatggtgcac tctcagtaca atctgctctg 3660
atgccgcata gttaagccag ccccgacacc cgccaacacc cgctgacgcg ccctgacggg 3720
cttgtctgct cccggcatcc gcttacagac aagctgtgac cgtctccggg agctgcatgt 3780
gtcagaggtt ttcaccgtca tcaccgaaac gcgcgagacg aaagggcctc gtgatacgcc 3840
tatttttata ggttaatgtc atgataataa tggtttctta gtatgatcca atatcaaagg 3900
aaatgatagc attgaaggat gagactaatc caattgagga gtggcagcat atagaacagc 3960
taaagggtag tgctgaagga agcatacgat accccgcatg gaatgggata atatcacagg 4020
aggtactaga ctacctttca tcctacataa atagacgcat ataagtacgc atttaagcat 4080
aaacacgcac tatgccgttc ttctcatgta tatatatata caggcaacac gcagatatag 4140
gtgcgacgtg aacagtgagc tgtatgtgcg cagctcgcgt tgcattttcg gaagcgctcg 4200
ttttcggaaa cgctttgaag ttcctattcc gaagttccta ttctctagaa agtataggaa 4260
cttcagagcg cttttgaaaa ccaaaagcgc tctgaagacg cactttcaaa aaaccaaaaa 4320
cgcaccggac tgtaacgagc tactaaaata ttgcgaatac cgcttccaca aacattgctc 4380
aaaagtatct ctttgctata tatctctgtg ctatatccct atataaccta cccatccacc 4440
tttcgctcct tgaacttgca tctaaactcg acctctacat tttttatgtt tatctctagt 4500
attactcttt agacaaaaaa attgtagtaa gaactattca tagagtgaat cgaaaacaat 4560
acgaaaatgt aaacatttcc tatacgtagt atatagagac aaaatagaag aaaccgttca 4620
taattttctg accaatgaag aatcatcaac gctatcactt tctgttcaca aagtatgcgc 4680
aatccacatc ggtatagaat ataatcgggg atgcctttat cttgaaaaaa tgcacccgca 4740
gcttcgctag taatcagtaa acgcgggaag tggagtcagg ctttttttat ggaagagaaa 4800
atagacacca aagtagcctt cttctaacct taacggacct acagtgcaaa aagttatcaa 4860
gagactgcat tatagagcgc acaaaggaga aaaaaagtaa tctaagatgc tttgttagaa 4920
aaatagcgct ctcgggatgc atttttgtag aacaaaaaag aagtatagat tctttgttgg 4980
taaaatagcg ctctcgcgtt gcatttctgt tctgtaaaaa tgcagctcag attctttgtt 5040
tgaaaaatta gcgctctcgc gttgcatttt tgttttacaa aaatgaagca cagattcttc 5100
gttggtaaaa tagcgctttc gcgttgcatt tctgttctgt aaaaatgcag ctcagattct 5160
ttgtttgaaa aattagcgct ctcgcgttgc atttttgttc tacaaaatga agcacagatg 5220
cttcgttcag gtggcacttt tcggggaaat gtgcgcggaa cccctatttg tttatttttc 5280
taaatacatt caaatatgta tccgctcatg agacaataac cctgataaat gcttcaataa 5340
tattgaaaaa ggaagagtat gagtattcaa catttccgtg tcgcccttat tccctttttt 5400
gcggcatttt gccttcctgt ttttgctcac ccagaaacgc tggtgaaagt aaaagatgct 5460

gaagatcagt tgggtgcacg agtgggttac atcgaactgg atctcaacag cggtaagatc 5520
cttgagagtt ttcgccccga agaacgtttt ccaatgatga gcacttttaa agttctgcta 5580
tgtggcgcgg tattatcccg tattgacgcc gggcaagagc aactcggtcg ccgcatacac 5640
tattctcaga atgacttggt tgagtactca ccagtcacag aaaagcatct tacggatggc 5700
atgacagtaa gagaattatg cagtgctgcc ataaccatga gtgataacac tgcggccaac 5760
ttacttctga caacgatcgg aggaccgaag gagctaaccg cttttttgca caacatgggg 5820
gatcatgtaa ctcgccttga tcgttgggaa ccggagctga atgaagccat accaaacgac 5880
gagcgtgaca ccacgatgcc tgtagcaatg gcaacaacgt tgcgcaaact attaactggc 5940
gaactactta ctctagcttc ccggcaacaa ttaatagact ggatggaggc ggataaagtt 6000
gcaggaccac ttctgcgctc ggcccttccg gctggctggt ttattgctga taaatctgga 6060
gccggtgagc gtgggtctcg cggtatcatt gcagcactgg ggccagatgg taagccctcc 6120
cgtatcgtag ttatctacac gacggggagt caggcaacta tggatgaacg aaatagacag 6180
atcgctgaga taggtgcctc actgattaag cattggtaac tgtcagacca agtttactca 6240
tatatacttt agattgattt aaaacttcat ttttaattta aaaggatcta ggtgaagatc 6300
ctttttgata atctcatgac caaaatccct taacgtgagt tttcgttcca ctgagcgtca 6360
gaccccgtag aaaagatcaa aggatcttct tgagatcctt tttttctgcg cgtaatctgc 6420
tgcttgcaaa caaaaaaacc accgctacca gcggtggttt gtttgccgga tcaagagcta 6480
ccaactcttt ttccgaaggt aactggcttc agcagagcgc agataccaaa tactgtcctt 6540
ctagtgtagc cgtagttagg ccaccacttc aagaactctg tagcaccgcc tacatacctc 6600
gctctgctaa tcctgttacc agtggctgct gccagtggcg ataagtcgtg tcttaccggg 6660
ttggactcaa gacgatagtt accggataag gcgcagcggt cgggctgaac ggggggttcg 6720
tgcacacagc ccagcttgga gcgaacgacc tacaccgaac tgagatacct acagcgtgag 6780
ctatgagaaa gcgccacgct tcccgaaggg agaaaggcgg acaggtatcc ggtaagcggc 6840
agggtcggaa caggagagcg cacgagggag cttccagggg gaaacgcctg gtatctttat 6900
agtcctgtcg ggtttcgcca cctctgactt gagcgtcgat ttttgtgatg ctcgtcaggg 6960
gggcggagcc tatggaaaaa cgccagcaac gcggcctttt tacggttcct ggccttttgc 7020
tggccttttg ctcacatgtt ctttcctgcg ttatcccctg attctgtgga taaccgtatt 7080
accgcctttg agtgagctga taccgctcgc cgcagccgaa cgaccgagcg cagcgagtca 7140
gtgagcgagg aagcggaaga gcgcccaata cgcaaaccgc ctctccccgc gcgttggccg 7200
attcattaat gcagctgaat tggagcgacc tcatgctata cctgagaaag caacctgacc 7260
tacaggaaag agttactcaa gaataagaat tttcgtttta aaacctaaga gtcactttaa 7320
aatttgtata cacttatttt ttttataact tatttaataa taaaaatcat aaatcataag 7380
aaattcgctt atttagaagt gtcaacaacg tatctaccaa cgatttgacc cttttccatc 7440
ttttcgtaaa tttctggcaa ggtagacaag ccgacaacct tgattggaga cttgaccaaa 7500
cctctggcga agaattgtta attaagagct cagatcttat cgtcgtcatc cttgtaatcc 7560
atcgatacta gtgcggccgc cctttagtga gggttgaatt cgaattttca aaattcttta 7620
cttttttttt ggatggacgc aaagaagttt aataatcata ttacatggca ttaccaccat 7680
atacatatcc atatacatat ccatatctaa tcttacttat atgttgtgga aatgtaaaga 7740
gccccattat cttagcctaa aaaaaccttc tctttggaac tttcagtaat acgcttaact 7800
gctcattgct atattgaagt acggattaga agccgccgag cgggtgacag ccctccgaag 7860
gaagactctc ctccgtgcgt cctcgtcttc accggtcgcg ttcctgaaac gcagatgtgc 7920
ctcgcgccg

<210> 2
<211> 35
<212> DNA
<213> Artificial sequence

<223> Oligo I, direct oligonucleotide primer used for generating the DNA
      fragment encoding pVP2-456 protein in combination with SEQ. ID. NO: 3

<400> 2
gcgcagatct atgacaaacc tgtcagatca aaccc                    35

<210> 3
<211> 32
<212> DNA
<213> Artificial sequence

<223> Oligo II, reverse oligonucleotide primer used for generating the DNA
      fragment encoding pVP2-456 protein in combination with SEQ. ID. NO: 2

<400> 3
gcgcaagctt acacagctat cctccttatg gc                                32

<210> 4
<211> 32
<212> DNA
<213> Artificial sequence

<223> Reverse oligonucleotide primer used for generating the DNA fragment
      encoding pVP2-441 protein in combination with SEQ. ID. NO: 2

<400> 4
gcgcaagctt ttatgctcct gcaatcttca gg                                32

<210> 5
<211> 40
<212> DNA
<213> Artificial sequence

<223> Reverse oligonucleotide primer used for generating the DNA fragment
      encoding pVP2-452 protein in combination with SEQ. ID. NO: 2

<400> 5
gcgcaagctt accttatggc ccggattatg tctttgaagc                        40

<210> 6
<211> 31
<212> DNA
<213> Artificial sequence

<223> Reverse oligonucleotide primer used for generating the DNA fragment
      encoding pVP2-466 protein in combination with SEQ. ID. NO: 2

<400> 6
gcgcaagctt aggcaggtgg gaacaatgtg g                                 31

<210> 7
<211> 33
<212> DNA
<213> Atificial sequence

<223> Reverse oligonucleotide primer used for generating the DNA fragment
      encoding pVP2-476 protein in combination with SEQ. ID. NO: 2

<400> 7
gcgcaagctt aaccttcccc aattgcatgg ggc                               33

<210> 8
<211> 33
<212> DNA

<213> Atificial sequence

<223> Reverse oligonucleotide primer used for generating the DNA fragment
      encoding pVP2-487 protein in combination with SEQ. ID. NO: 2

<400> 8
gcgcaagctt aggcctgggc ctcatcgccc agc                              33


<210> 9
<211> 32
<212> DNA
<213> Atificial sequence

<223> Reverse oligonucleotide primer used for generating the DNA fragment
      encoding pVP2-494 protein in combination with SEQ. ID. NO: 2

<400> 9
gcgcaagctt aggctcgagc agttcccgaa gc                               32


<210> 10
<211> 32
<212> DNA
<213> Atificial sequence

<223> Reverse oligonucleotide primer used for generating the DNA fragment
      encoding pVP2-501 protein in combination with SEQ. ID. NO: 2

<400> 10
gcgcaagctt aaggtcttgc ttttcctgac gc                               32


<210> 11
<211> 34
<212> DNA
<213> Atificial sequence

<223> Reverse oligonucleotide primer used for generating the DNA fragment
      encoding pVP2-512 protein in combination with SEQ. ID. NO: 2

<400> 11
gcgcaagctt aggcgagagt cagctgcctt atgc                             34